# EUROPEAN PATENT APPLICATION

(11) **EP 4 578 479 A1**
(43) Date of publication of application: **02.07.2025**
(21) Application number: 23857682.1
(22) Date of filing: 22.08.2023
(51) Int. Cl.: A61M 25/00, A61M 25/01, A61M 25/02

(54) **FOLEY CATHETER SUPPORT TUBE ASSEMBLY**

(30) Priority: 24.08.2022 KR 20220106212; 02.09.2022 KR 20220111505; 10.02.2023 KR 20230017999
(71) Applicant: Ufuhealth Co, Seoul 06022 (KR)
(72) Inventor: KIM, Mi Yon, Seoul 06057 (KR)
(74) Representative: RGTH
(86) International application number: PCT/KR2023/012365
(87) International publication number: WO 2024/043650

(57) **Abstract**

The present application relates to: a driving module provided in a support tube for guiding a Foley catheter when the Foley catheter is inserted; and a Foley catheter support tube assembly in which the driving module is provided, wherein the driving module includes: a first part including side surfaces, which have a pair of first roller insertion holes in which a first roller is provided, and a connecting surface between the side surfaces; a second part which is disposed at the connecting surface of the first part, aligned with the pair of first roller insertion holes, and which has a pair of second roller insertion holes in which a second roller is provided; third parts each being arranged at respective side surfaces of the first part, and having a pair of half guide-holes for guiding the Foley catheter; and a fourth part disposed at the connecting surface of the first part so as to be positioned in a space formed by the third parts.

## Description

### [Technical Field]

The present invention relates to a Foley catheter support tube assembly.

### [Background Art]

Artificial urination refers to insertion of a catheter into the bladder through the urethra to drain urine to the outside, and a Foley catheter is used when the catheter needs to be maintained continuously for a certain period of time. Artificial urination using a Foley catheter is widely used to drain urine or measure an amount of urine in critical patients, general surgery patients, urological surgery patients, or other patients with difficulty urinating.

However, a process of inserting a Foley catheter into the bladder includes cumbersome processes in preparation and procedure stages. In a hospital, a Foley catheter set must be prepared in advance, and the Foley catheter set requires aseptic treatment and includes a sterile dressing, forceps, a Kelly clamp, and three containers for lubricating jelly, a saline solution for a ballon, and disinfectant cotton. In the procedure preparation stage, in order to perform the procedure, medical personnel must open the Foley catheter set, separate a Foley catheter and a ballon syringe from packaging, add the lubricating jelly, the saline solution for the ballon, and the disinfectant cotton into the corresponding containers, and pay special attention to prevent contamination during each process, and this stage is cumbersome and time-consuming. In terms of hospital management, such Foley catheter set preparation and procedure preparation processes lead to increased costs and waste of labor of highly skilled medical personnel. Therefore, it may be said that development of a device and method that may simplify the preparation processes is required.

For this purpose, an assembly disclosed in Korean patent registration No. 10-1058241 entitled "Assembly for Foley catheter insertion" has been used.

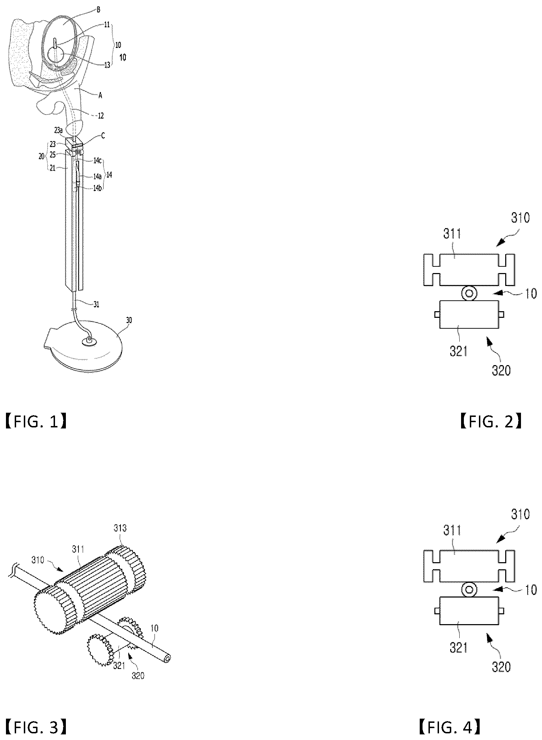

FIG. 1 is an exemplary view illustrating a state in which a Foley catheter 10 is inserted into the bladder B through a Foley catheter assembly 20. The Foley catheter assembly 20 has an advantage in that contamination is minimized before inserting the Foley catheter 10 into the bladder and a procedure process by an operator is simplified. However, the Foley catheter assembly 20 has a disadvantage in that it is a disposable product used to insert the Foley catheter 1 into the human body and is discarded after use, but has high manufacturing costs.

That is, the conventional Foley catheter assembly 20 has the disadvantage of high manufacturing costs because it is manufactured by injection molding or the like using a synthetic resin, such as plastic. Therefore, there is a disadvantage of increased material procurement costs for hospitals using the Foley catheter assembly 20.

The Foley catheter 10 is disposed between a first roller 310 and a second roller 320, and moves forward due to frictional force between the rollers and the Foley catheter when the first roller 310 and the second roller 320 rotate. At this time, based on the driving principle of the Foley catheter assembly, the Foley catheter moves forward only by the rotation of the first roller at the top (i.e., a structure in which the rotational force of the first roller is transmitted to the second roller through the Foley catheter is adopted), and in this case, there is a problem in which, even if there is slight resistance to the forward movement of the Foley catheter, the second roller at the bottom does not rotate and thus the Foley catheter does not move forward. In addition, the Foley catheter 10 is manufactured using a soft material having designated elasticity due to the characteristics thereof, and thus, if one side of a roller operation knob is pressed and turned, the catheter moves forward while being deflected to the left or right instead of the center when the first roller rotates. In addition, there is a disadvantage in that, when the Foley catheter is deflected to the maximum deflection position, even if the first roller rotates, friction between the Foley catheter and a housing causes only the roller to idle, and does not provide sufficient frictional force to move the catheter forward so that the catheter does not move any further forward.

(Patent Document 1) Korean Patent Registration No. 10-1058241 (August 12, 2011)
(Patent Document 2) Korean Patent Laid-open Publication No. 10-2022-0138265 (October 12, 2022)

### [Disclosure]

### [Technical Problem]

Therefore, the present invention has been made in view of the above problems, and it is an object of the present invention to provide a Foley catheter support tube assembly that includes a foldable support tube having the same function as conventional Foley catheter support tubes and manufactured using a foldable material (paper, vinyl, or a synthetic resin material) so as to reduce manufacturing costs, and particularly, has improved accuracy in a procedure due to the high strength of a driving module that guides a Foley catheter.

It is another object of the present invention to provide a driving unit in which an alignment groove is formed on rollers so as to prevent deflection of a Foley catheter.

### [Technical Solution]

In order to solve the above objects, one embodiment of the present invention provide a driving module for Foley catheter support assemblies installed in a support tube configured to guide a Foley catheter when inserting the Foley catheter, including a first part including side surfaces provided with a pair of first roller insertion holes configured such that a first roller is installed therein, and a connection surface between the side surfaces, a second part disposed on the connection surface of the first part aligned with the pair of first roller insertion holes, and provided with a pair of second roller insertion holes configured such that a second roller is installed therein, third parts disposed on the side surfaces of the first part, respectively, and provided with a pair of half guide holes configured to guide the Foley catheter, and a fourth part disposed on the connection surface of the first part so as to be located within a space formed by the third parts.

In one embodiment, a distance from the connection surface of the first part to the first roller insertion holes may be longer than a distance from the connection surface of the first part to the second roller insertion holes.

In one embodiment, a difference between the distance from the connection surface of the first part to the first roller insertion holes and the distance from the connection surface of the first part to the second roller insertion holes may correspond to a thickness of the Foley catheter.

In one embodiment, each of the third parts may include an upper surface provided with one of the pair of half guide holes, and a lower surface spaced apart from the upper surface and provided with the other of the pair of half guide holes.

In one embodiment, a direction in which the pair of first roller insertion holes of the first part face each other and a direction in which the pair of half guide holes of the third parts face each other may intersect each other.

In one embodiment, a jelly accommodation space surrounded by the upper surface and the lower surface of each of the third parts and the side surfaces of the first part may be formed by folding connection portions between the side surfaces and the connection surface of the first part in a direction in which the third parts approach each other.

In one embodiment, leakage prevention parts may be formed on contact surfaces of the third parts provided on the side surfaces of the first part in a longitudinal direction of the contact surfaces.

In one embodiment, a coupling part may be formed on at least one of the leakage prevention parts so as to be coupled to the other of the leakage prevention parts.

In one embodiment, the fourth part may have a predetermined height and be disposed in the jelly accommodation space.

In one embodiment, a guide groove configured to extend in an insertion direction of the Foley catheter may be formed on the fourth part.

In one embodiment, the half guide hole formed on the upper surface may be disposed closer to the connection surface of the first part than the half guide hole formed on the lower surface.

In one embodiment, the half guide hole formed on the lower surface may be disposed closer to the connection surface of the first part than the half guide hole formed on the upper surface.

In one embodiment, gear teeth may be formed on an outer surfaces of the first roller and the second roller, and an alignment groove may be formed on the outer surface of the first roller or the second roller.

In one embodiment, the alignment groove may have a depressed portion from the outer surface of the first roller or the second roller in a direction away from the Foley catheter.

In one embodiment, a distance from the first roller or the second roller to the alignment groove may correspond to a thickness of the Foley catheter.

In one embodiment, the Foley catheter may be manufactured using a soft material.

In addition, the present invention provides a Foley catheter support tube assembly provided with the above-described driving module installed therein and including a front surface, a rear surface, a first side surface, and a second side surface, wherein an open path configured such that a fluid injection tube of the Foley catheter passes therethrough is formed in any one of the front surface, the rear surface, the first side surface, and the second side surface, and a pair of first roller coupling holes aligned with the pair of first roller insertion holes of the first part is formed in surfaces located on both sides of the surface provided with the open path formed therein.

In one embodiment, first and second syringe accommodation part cutting lines configured to traverse at least three surfaces other than the surface provided with the open path formed therein may be further formed, and syringe cover surfaces may be formed to be sequentially arranged in parallel on one side of an attachment surface arranged parallel to the front surface.

In one embodiment, a syringe accommodation part may be formed by cutting the first and second syringe accommodation part cutting lines and folding connection portions between the three surfaces, and the syringe cover surfaces may be folded to surround an outer surface of the syringe accommodation part.

In one embodiment, an upper cutting line and a side lower cutting line configured to traverse the front surface, the rear surface, the first side surface, and the second side surface may be further formed and, on both side surfaces connected to a surface opposite to the surface provided with the open path formed therein, first support cutting lines bent so that one side of each thereof is connected to the upper cutting line and the other side of each thereof is connected to a corresponding one of connection portions between the opposite surface and the side surfaces, and second support cutting lines bent so that one side of each thereof is connected to the side lower cutting line and the other side of each thereof is connected to a corresponding one of the connection portions between the opposite surface and the side surfaces may be formed.

In one embodiment, supports configured to support the support tube assembly may be formed by cutting the upper cutting line, the side lower cutting line, the first support cutting lines, and the second support cutting lines.

In one embodiment, an open path cutting line cut to form the open path may be formed on any one of the front surface, the rear surface, the first side surface, and the second side surface, and an open path exposure cutting line configured to expose a portion of the open path cutting line may be further formed on the surface.

In one embodiment, incision parts may be formed in an oblique direction inside the open path cutting line, and the oblique direction may a direction inclined opposite to a direction in which the open path cutting line is cut.

In one embodiment, a clip cutting line configured to install a clip therein may be formed on each of three surfaces other than the surface provided with the open cutting line formed thereon.

### [Advantageous effects]

The present invention as described above has the following effects.

A foldable support tube may be manufactured using a foldable material (paper, vinyl, or a synthetic resin material) while providing the same function as conventional Foley catheter support tubes, thereby being capable of reducing manufacturing costs.

A driving module that provides driving force to guide a Foley catheter is manufactured using a material having predetermined rigidity, and is installed in the foldable support tube, thereby improving durability of a support tube assembly and accuracy of a procedure.

The support tube assembly in a state in which the upper and lower parts of the support tube assembly are closed by a bottom surface and a cover surface before use is provided to a medical professional, and the bottom surface and the cover surface are removed just before the procedure is performed on a person receiving the procedure, thereby being capable of being easily stored and transported, and maintaining sterility for a long period of time.

A reverse rotation prevention structure for transportation rollers that guide the Foley catheter into the bladder is applied, thereby preventing the Foley catheter from being separated between a main roller and a sub-roller during storage, and solving separation of the Foley catheter inserted into the body of the person receiving the procedure from the body at the time of the procedure.

In addition, a backward movement prevention part configured to prevent the Foley catheter from moving backward is applied, thereby preventing the Foley catheter from being separated between the main roller and the sub-roller during storage, and solving separation of the Foley catheter inserted into the body of the person receiving the procedure from the body due to backward movement of the Foley catheter at the time of the procedure.

In addition, a medical supplies accommodation part that may store various medical supplies (a syringe, disinfectant cotton, a gel, distilled water, etc.) required during the procedure of inserting the Foley catheter into the bladder are provided, thereby enabling the procedure to be simply performed without a process of preparing separate medical supplies required for the procedure by a medical professional.

In addition, a component that guides the Foley catheter to be inserted in the vertical direction in the case of a male and guides the Foley catheter to be inserted in the horizontal direction in the case of a female is provided, thereby being capable of ensuring ease of the procedure by the medical professional due to a difference in the genital structure depending on gender.

In addition, an alignment groove is formed on the rollers that provide force to transport a catheter tube so that the catheter tube receives force simultaneously by rotation of first and second rollers and, when driven, the catheter tube is inserted into the human body while overcoming resistance applied to the catheter tube, thereby enabling the catheter tube to move forward and backward without being deflected to one side, and thus reducing pain of the person receiving the procedure and ensuring convenience of the procedure.

In addition, the lower end of the Foley catheter is supported by a fourth part and a fifth part, and the upper end of the Foley catheter is pressed by a pressing element, thereby enabling the Foley catheter to be inserted into a correct position without being deflected in every direction.

In addition, a clip having a predetermined strength is installed on the foldable support tube, thereby being capable of preventing the shape of the foldable support tube from being deformed due to the weight of components required for the Foley catheter procedure.

### [Description of Drawings]

FIG. 1 is a schematic view illustrating a conventional support tube assembly.
FIG. 2 is a schematic view illustrating rollers that provide force to transport a Foley catheter in a conventional device.
FIGs. 3 to 6 are schematic views illustrating a driving unit that provides force to transport a Foley catheter according to embodiments of the present invention.
FIG. 7 is a view illustrating a driving module that constitutes a support tube assembly according to one embodiment of the present invention.
FIG. 8 is a view illustrating a coupling structure of leakage prevention parts that prevent external leakage of gel from the driving module of FIG. 7.
FIG. 9 is a view specifically illustrating a fourth part of the driving module of FIG. 7.
FIG. 10 is a development view of a foldable support tube that constitutes the support tube assembly according to one embodiment of the present invention.
FIG. 11 is a view illustrating coupling of the rollers to the foldable support tube formed by folding the developed form thereof shown in FIG. 10.
FIG. 12 is a view illustrating installation of the driving module of FIG. 7 in the foldable support tube of FIG 10.
FIG. 13 is a view illustrating the support tube assembly, the installation of which has been completed according to FIG. 12.
FIG. 14 is a view illustrating application of gel accommodated in a gel accommodation space to the Foley catheter while the Foley catheter is guided by the rollers.
FIG. 15 is a view illustrating a roller reverse rotation prevention structure according to one embodiment of the present invention.
FIG. 16 is a view illustrating the state of use of the Foley catheter inserted into the support tube assembly.
FIG. 17 is a development view of another foldable support tube additionally provided with a syringe accommodation part.
FIGs. 18 and 19 are views illustrating installation of a syringe in the foldable support tube of FIG. 17.
FIG. 20 is a view illustrating a backward movement prevention part according to one embodiment of the present invention.
FIG. 21 is a development view illustrating a foldable support tube that constitutes a support tube assembly according to another embodiment of the present invention.
FIG. 22 is a development view illustrating a foldable support tube that constitutes a support tube assembly according to yet another embodiment of the present invention.
FIG. 23 is a view illustrating a clip that may be mounted on the foldable support tube of FIG. 22.

### [Mode for Invention]

In some cases, in order to avoid ambiguity in the concept of the present invention, known structures and devices will be omitted or illustrated in a block diagram form based on the core functions of each structure and device.

Throughout the following description, when a part is said to "comprise" or "include" a component, it does not exclude other components, and may further include other components, unless the context clearly indicates otherwise. In addition, in the following description of the embodiments, it will be understood that the terms "...part," "...unit," "module," etc. indicate units for processing at least one function or operation, and may be implemented as software, hardware, or a combination of software and hardware. In addition, as used herein, "a" or "an," "one," "the," and similar related terms may be used in the context of describing the present invention (particularly, in the context of the claims below) to include both singular and plural meanings, unless indicated otherwise in the description or clearly contradicted by the content.

In the following description of embodiments of the present invention, a detailed description of known functions and configurations incorporated herein will be omitted when it may make the subject matter of the present invention rather unclear. Also, the terms used in the following description are terms defined taking into consideration the functions in the embodiments of the present invention, and may be changed depending on the intention of a user or operation or the custom. Therefore, the definitions of these terms should be determined based on the whole content of the following description.

Hereinafter, the present invention will be described in detail with reference to the accompanying drawings.

First, a driving unit according to one embodiment of the present invention will be described in detail with reference to FIGs. 2 to 6.

The driving unit according to one embodiment of the present invention includes a first roller 310 and a second roller 320, and a Foley catheter 10 is disposed between the first roller 310 and the second roller 320 and may move forward by frictional force generated between the rollers and the Foley catheter due to rotation of the first roller 310 and the second roller 320.

FIG. 2 illustrates a conventional driving unit. Referring to FIG. 2, a main body 311 of a first roller and a main body 321 of a second roller are provided in a cylindrical shape. In addition, the Foley catheter 10 is structured to be disposed between the cylindrical main bodies.

At the time of a Foley catheter insertion procedure, a medical professional moves the Foley catheter 10 forward by rotating the first roller or the second roller while holding the same with one hand. The rotational force of the first roller or the second roller is transmitted to the Foley catheter 10, and force that moves the Foley catheter 10 is transmitted to the second roller or the first roller, thereby enabling the first roller and the second roller to rotate together. However, when the roller is rotated with one hand, the force is inevitably biased to the left or right, and the Foley catheter 10 disposed between the rollers moves forward while being deflected to the left or right accordingly. In addition, if the Foley catheter 10 moves forward to the maximum deflection position, even if the roller is rotated, the Foley catheter 10 does not move any further forward and the roller idles.

The driving unit according to one embodiment of the present invention to solve the above-described problem is illustrated in FIGs. 3 to 6.

In the same manner as FIG. 2, the first roller 310 and the second roller 320 are provided, and the Foley catheter 10 is disposed between the rollers. However, while the main bodies of the rollers shown in FIG. 2 are cylindrical, an alignment groove 311a, 311b, 321a or 321b is formed in a first roller main body 311 or a second roller main body 321 shown in any of FIGs. 3 to 6.

A plurality of gear teeth is formed in the outer surfaces of the first roller 310 and the second roller 320. The gear teeth of the first roller 310 and the gear teeth of the second roller 320 are coupled to be engaged with each other. Therefore, even if only one of the first roller 310 and the second roller 320 is operated, both rollers rotate.

The alignment groove 321a or 321b is provided in a depressed form on the center of the main body 311 or 321 of each roller 310 or 320. Specifically, the alignment groove 321a or 321b is provided in a depressed form from the outer surface of the first roller 310 or the second roller 320 in a direction away from the Foley catheter 10. The shape of the alignment groove may include one of those shown in FIGs. 3 to 6, but is not particularly limited thereto. Although FIGs. 3 to 6 illustrate the alignment groove 321a or 321b formed in the second roller, an embodiment in which an alignment groove is formed in the first roller is also within the scope of the present invention.

Furthermore, a distance from the center of the first roller main body 311 to the alignment groove and a distance d from the center of the second roller main body 321 to the alignment groove 311a or 311b may correspond to the thickness (diameter) of the Foley catheter 10. Specifically, the distance d may be the same as the diameter of the Foley catheter 10, and preferably, the distance may be smaller than the diameter of the Foley catheter 10. As a result, even if the Foley catheter 10 is disposed in the alignment groove, the contact area between the rollers and the Foley catheter is increased, so that the frictional force due to rotation of the roller may be completely transmitted to the Foley catheter 10. Therefore, the Foley catheter 10 may move forward and be inserted into the human body even with a small amount of force.

In addition, the width of the alignment groove 321a or 321b may also correspond to the thickness (diameter) of the Foley catheter 10. Specifically, the width may be the same as the diameter of the Foley catheter 10, and preferably, the width may be greater than the diameter of the Foley catheter 10. Because the larger width means that a space in which the Foley catheter 10 is deflected to the left or right becomes wider, the width may be 1 to 1.5 times the diameter of the Foley catheter 10.

The gear teeth of the roller main body in a section in which the alignment groove 321a or 321b is not formed may be engaged with the gear teeth of the other roller main body. Thereby, the Foley catheter 10 is disposed in the alignment groove 321a or 321b, which is a closed space, and even if the roller is rotated, the Foley catheter 10 is deflected only within the closed space, and may move forward while being generally located in the center of the roller main body. Consequently, the pain of a person receiving the procedure is minimized, and difficulty of the procedure is also reduced.

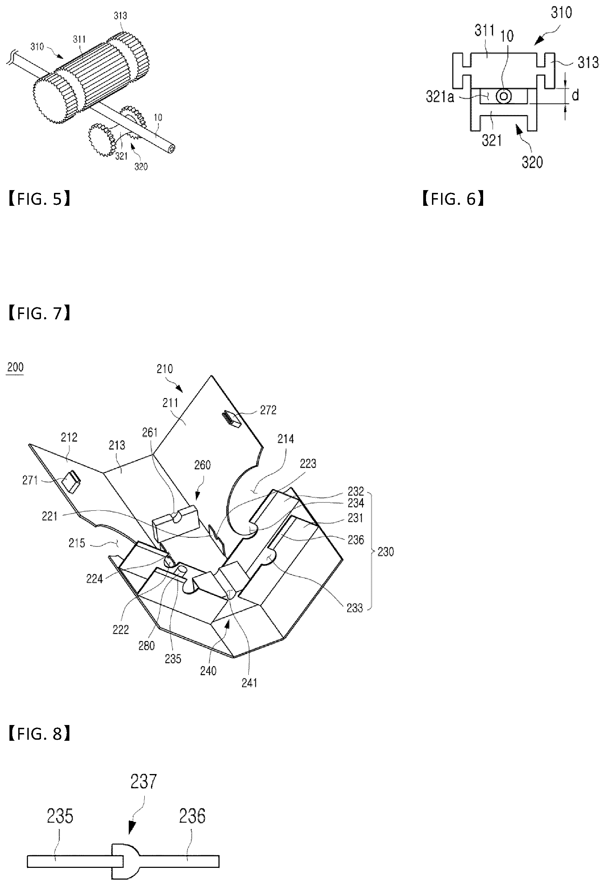

Next, a driving module 200 according to one embodiment of the present invention is specifically described with reference to FIGs. 7 to 9. The above-described driving unit is installed in the driving module 200 according to one embodiment of the present invention, and the driving module 200 may be manufactured using a material that is inexpensive, has a certain strength, and is easy to mold, such as plastic, but is not particularly limited thereto. That is, the driving module 200 serves as a support in which the driving unit is installed and which supports the installed driving unit.

The driving module 200 in which the driving unit according to one embodiment of the present invention is installed in a foldable support tube 100, which will be described below, and provides driving force to insert a catheter tube 12 of the Foley catheter 10 into the genital organ of the person receiving the procedure, specifically, an insertion tip 11 of the Foley catheter 10 into the bladder.

Referring to FIG. 7, the driving module 200 includes a first part 210, a second part 220, third parts 230, and a fourth part 240. The respective parts may be integrally provided to form the driving module 200, but in other embodiments, they may be provided in separate elements and assembled to form the driving module 200.

The first part 210 includes side surfaces 211 and 212 in which a pair of first roller insertion holes 214 and 215 in which the first roller 310 is installed is formed, and a connection surface 213 between the side surfaces 211 and 212. The pair of first roller insertion holes 214 and 215 may be formed in a form that is depressed inward from the outermost edges of the side surfaces 211 and 212 (toward the connection surface).

The first roller 310 is installed to pass through a pair of first roller coupling holes 123 and 143 of the foldable support tube 100, which will be described below, and the pair of first roller insertion holes 214 and 215 of the driving module 200.

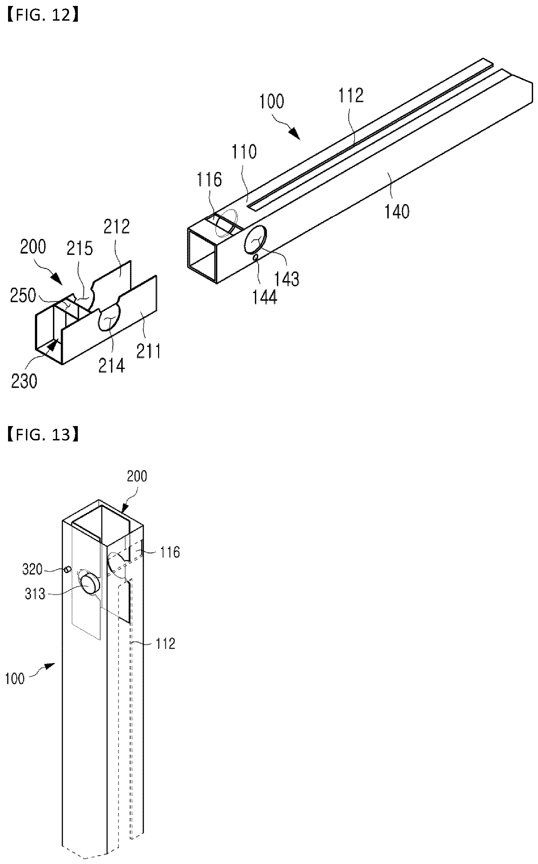

An insertion protrusion 271 is formed on one side surface 212 among the side surfaces 211 and 212 so as to be coupled to an insertion groove 272 formed on the other side surface 211. If the side surfaces 211 and 212 move in directions approaching each other so that the insertion protrusion 271 is coupled to the insertion groove 272, the folded form of the driving module 200 may be fixed. Assembly of a support tube assembly may be achieved by inserting the driving module 200 in a state in which the folded form of the driving module 200 is fixed into the foldable support tube 100 shown in FIG. 12.

The second part 220 is disposed on the connection surface 213 of the first part 210, and includes side surfaces 221 and 222 in which a pair of second roller insertion holes 223 and 224 in which the second roller 320 is installed is formed. The pair of second roller insertion holes 223 and 224 may be formed in a form that is depressed inward from the outermost edges of the side surfaces 221 and 222 (toward the connection surface). In another example, the second roller insertion holes 223 and 224 may be formed through the side surfaces 211 and 212 of the first part 210.

The second roller 320 may be installed to pass through a pair of second roller coupling holes 124 and 144 of the foldable support tube 100, which will be described below, and the pair of second roller insertion holes 223 and 224 of the driving module 200, and in another example, may be installed to be seated in the second roller insertion holes 223 and 224.

Here, a distance from the connection surface 213 to the first roller insertion holes 214 and 215 is longer than a distance from the connection surface 213 to the second roller insertion holes 223 and 224, and a difference therebetween may correspond to the thickness of the catheter tube 12. The catheter tube 12 is located between the first roller 310 and the second roller 320, the catheter tube 12 moves forward and backward while rotating together with the second roller 320 through operation of the first roller 310 (specifically, operation of an operation knob), and because the difference has a value corresponding to the thickness of the catheter tube 12, the rotational force of the first roller 310 and the second roller 320 may be intactly transmitted to the catheter tube 12. Therefore, an advantage of being capable of performing the Foley catheter insertion procedure with a small force is achieved.

The third parts 230 may be provided as a pair, and one of the pair of third parts 230 is disposed on the side surface 211 of the first part 210 and the other is disposed on the other side surface 212 of the first part 210.

Hereinafter, one third part 230 will be descried as an example.

The third part 230 is provided with a pair of half guide holes 233 and 234 to guide the catheter tube 12, and includes an upper surface 231 and a lower surface 232 in which the pair of half guide holes 233 and 234 is formed.

The upper surface 231 and the lower surface 232 are spaced apart from each other in the travel direction of the catheter tube 12, and the half guide hole 233 formed in the upper surface 231 and the half guide hole 234 formed in the lower surface 232 are aligned in the travel direction.

The pair of third parts 230 may be arranged to face each other, and connected to each other, thereby enabling the two half guide holes to form a pair of complete guide holes. The catheter tube 12 passes through these guide holes.

When inserting the Foley catheter, a subject for the procedure is placed in a lying position to receive the procedure. Due to the characteristics of the human body structure depending on gender, the catheter should be inserted into a male subject in the vertical direction, and the catheter should be inserted into a female subject in the horizontal direction.

Therefore, at the time of the Foley catheter insertion procedure, in the case of a male subject, the Foley catheter should face downward so that an operator may easily grasp the foldable support tube 100 to insert the catheter, and in the case of a female subject, the Foley catheter should face horizontally so that the operator may easily grasp the foldable support tube 100 to insert the catheter.

Therefore, the insertion angle of the catheter may be adjusted by changing the positions of the half guide holes formed in the upper surfaces 231 and the half guide holes formed in the lower surfaces 232 of the third parts 230.

Specifically, in one embodiment of the present invention, the half guide hole 233 formed in the upper surface 231 is located lower than the half guide hole 234 formed in the lower surface 232 (i.e., located closer to the connection surface of the first part), so that, if the catheter faces downward while passing through the guide holes and the support tube assembly is held by the operator's hand, the Foley catheter 10 may be easily vertically inserted naturally due to the angle of the operator's wrist. The effect is maximized when performing the procedure on a male subject.

In addition, in another embodiment of the present invention, the half guide hole 233 formed in the upper surface 231 is located higher than the half guide hole 234 formed in the lower surface 232 (i.e., located farther away from the connection surface of the first part), so that, if the catheter faces upward while passing through the guide holes and the support tube assembly is held by the operator's hand, the Foley catheter 10 may be easily horizontally inserted naturally due to the angle of the operator's wrist. The effect is maximized when performing the procedure on a female subject.

Furthermore, the pair of third parts 230 may be disposed perpendicularly to the side surfaces 211 and 212 of the first part 210, and therefore, a direction in which the pair of main roller insertion holes 214 and 214 of the first part face each other and a direction in which the pair of guide holes of the third parts face each other may intersect each other (specifically, perpendicularly to each other). In addition, a gel accommodation space 250 surrounded by the upper surfaces 231 and the lower surfaces 232 of the third parts 230 and the side surfaces 211 and 212 of the first part 210 is formed by connecting the third parts to each other. The gel accommodation space 250 accommodates a gel, and the gel (jelly) is applied to the catheter tube 12 passing through the gel accommodation space 250. By applying the gel to the catheter tube 12, friction occurring between the catheter tube 12 and the bladder during the process of inserting the catheter tube 12 into the bladder may be minimized, thereby enabling the catheter tube 12 to be easily inserted into the bladder and minimizing pain of the person receiving the procedure.

Leakage prevention parts 235 and 236 are formed on some areas of contact surfaces of the third parts 230 that come into contact with each other. The leakage prevention parts 235 and 236 are formed in the longitudinal direction of the contact surfaces, and a coupling part 237 is formed on one leakage prevention part 236 so as to be coupled to the other leakage prevention part 235. For example, the coupling part 237 may be provided in the form of a hook having a recess formed in the center thereof, and coupling of the third parts 230 may be achieved by coupling the other leakage prevention part 235 to the recess. The sealing ability of the gel accommodation space 250 is strengthened due to the leakage prevention parts 235 and 236, so that external leakage of the gel accommodated in the gel accommodation space 250 may be prevented.

The fourth part 240 is disposed within the gel accommodation space 250 and has a predetermined height. The fourth part 240 is configured to secure the rigidity of the third parts 230. Connection portions between the side surfaces 211 and 212 and the connection surface 213 of the first part 210 may be provided to have a smaller thickness than other portions. By the thin connection portions, the side surfaces 211 and 212 may be folded in a direction in which the third parts 230 come closer to each other, thereby completing the shape of the driving module 200.

The fourth part 230 is disposed in alignment with the third parts 230 in the width direction, and is disposed within the gel accommodation space 250 formed by folding the side surfaces 211 and 212. Because the fourth part 240 has the predetermined height, it is possible to provide a designated durability/rigidity to the third parts 230.

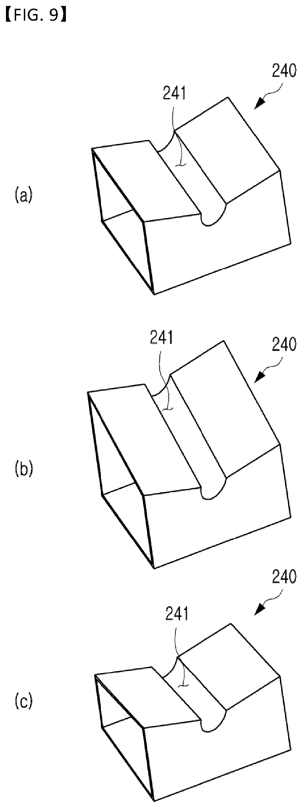

In addition, as shown in FIGs. 7 and 9, a guide groove 241 extending in the insertion direction of the catheter tube 12 is formed on the upper surface of the fourth part 240. The catheter tube 12 may be supported by the guide holes of the third parts 230 and the guide groove 241 of the fourth part 240.

Furthermore, the upper surface of the fourth part 240 may be formed to be inclined so that the height thereof decreases as the upper surface of the fourth part 240 gets closer to the guide groove 241. The fourth part 240 is disposed within the gel accommodation space 250. Because the height of the upper surface of the fourth part 240 decreases as the upper surface of the fourth part 240 gets closer to the guide groove 241, the gel in the gel accommodation space 250 may be concentrated into the guide groove 241, and thus, a larger amount of the gel may be applied to the catheter tube 12 passing through the guide groove 241.

The guide groove 241 of the fourth part 240 may be formed to be aligned with the guide holes of the third parts 230.

That is, in the case of an embodiment in which the height of the guide holes of the third parts 230 is decreased from the lower surface 232 to the upper surface 231 to facilitate catheter insertion for a male subject, the height of the guide groove 241 of the fourth part 240 may also be decreased from the lower surface 232 to the upper surface 231 (see FIG. 9(b)).

In addition, in the case of an embodiment in which the height of the guide holes of the third parts 230 is increased from the lower surface 232 to the upper surface 231 to facilitate catheter insertion for a female subject, the height of the guide groove 241 of the fourth part 240 may also be increased from the lower surface 232 to the upper surface 231 (see FIG. 9(c)).

Further, the fourth part 240 is disposed in front of the first roller insertion holes 214 and 215 and the second roller insertion holes 223 and 224 in which the first roller 310 and the second roller 320 are installed, i.e., closer the person receiving the procedure than the rollers, as shown in FIG. 7.

The driving module 200 according to one embodiment of the present invention may further include a fifth part 260 disposed behind the first roller insertion holes 214 and 215 and the second roller insertion holes 223 and 224, i.e., farther away from the person receiving the procedure than the rollers.

A groove 261 to guide the Foley catheter 10 is formed on the fifth part 260, in the same manner as the fourth part 240, and the groove 261 of the fifth part 260 and the guide groove 241 of the fourth part 240 may be aligned in a row. Therefore, the Foley catheter 10 may be supported by the fourth part 240 and the fifth part 250 at areas located in front of and behind the rollers, and the grooves to guide movement of the Foley catheter 10 are formed on the fourth part and the fifth part, so that insertion of the Foley catheter 10 may be facilitated.

In addition, a pressing element 280 may be formed on one of the side surfaces 211 and 212. The pressing element 280 is formed to protrude at a position farther away from the connection surface 213 than the half guide holes 233 and 234. The lower portion of the Foley catheter 10 is supported by the fourth part 240, and the upper portion of the Foley catheter 10 is pressed by the pressing element 280, thereby being capable of preventing a problem in which the Foley catheter 10 is inserted toward an unintended position.

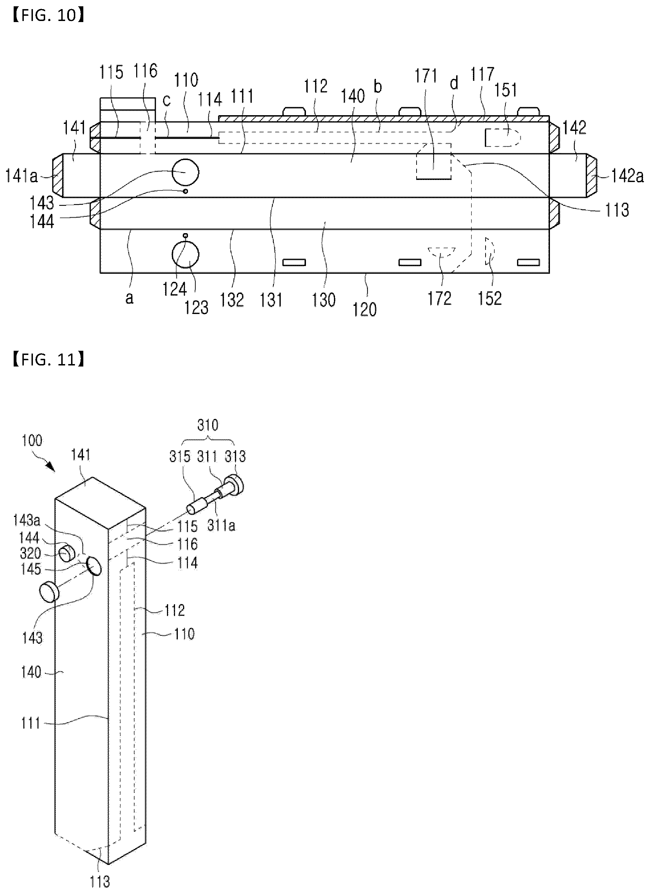

Next, the foldable support tube 100 to support the Foley catheter according to one embodiment will be described in detail with reference to FIG. 10.

The foldable support tube 100 to support the Foley catheter according to one embodiment of the present invention may be formed of a foldable material (e.g., paper, vinyl, or a synthetic resin material), and therefore has advantages in which less manufacturing costs are required, and a process of removing the foldable support tube 100 from the Foley catheter after the insertion procedure of the disposable Foley catheter is easy and simple.

The foldable support tube 100 according to one embodiment of the present invention is provided in the the form of the development view, as shown in FIG. 10, and a medical professional may fold each component along a folding line provided in the development view and cut some components along cutting lines to form a support tube assembly 1. In another embodiment, the foldable support tube 100 in a pre-folded state in which the Foley catheter is packaged may be provided to a medical professional.

Referring to FIG. 10, the foldable support tube 100 will be described in detail. The foldable support tube 100 shown in the development view of FIG. 10 serves as a support tube that guides the catheter tube 12 of the Foley catheter 10. Manufacture of the support tube assembly 1 may be completed by installing the driving module 200, which will be described below, in the foldable support tube 100.

The development view of the foldable support tube 100 according to one embodiment of the present invention includes a plurality of surfaces, i.e., a front surface 110, a rear surface 120, a first side surface 130, and a second side surface 140 that are arranged in parallel adjacent to each other. Here, the parallel arrangement means that, when the Foley catheter 10 is provided in the foldable support tube 100, the surfaces are arranged in a direction of traversing the Foley catheter 10, and serial arrangement means that the surfaces are arranged in a direction parallel to the Foley catheter 10.

The front surface 110, the rear surface 120, the first side surface 130, and the second side surface 140 may be folded by folding lines 111, 131, and 132 located on connection surfaces between the respective surfaces to form a rectangular tube having a rectangular cross section. The arrangement order of the front surface 110, the rear surface 120, the first side surface 130, and the second side surface 140 may be changed within the range in which the shape of a rectangular tube may be formed, and in other embodiments, support tubes having various polygonal tube shapes, such as a circular tube having a circular cross section, a pentagonal tube having a pentagonal cross section, and a hexagonal tube having a hexagonal cross section, may be formed.

In FIG. 10, a solid line marked as *a* represents a folding line, a dotted line marked as *b* represents a cutting line cut by a user (medical professional, or the like) before the procedure, a solid line marked as *c* represents a cutting line cut after insertion of the Foley catheter, and a hatched area marked as *d* represents an adhesive surface that is folded and adhered to another surface. The folding lines may be formed to have a smaller thickness than other areas, that is, the folding lines are formed to be depressed to a certain depth compared to other areas so that, when a user applies external force, surfaces located on both sides of each folding line are folded together and the folded state is maintained.

The front surface 110, the rear surface 120, the first side surface 130, and the second side surface 140 are folded with respect to each other to form the side surfaces of the support tube assembly 1.

The foldable support tube 100 according to one embodiment of the present invention may include a cover surface 141 and a bottom surface 142.

The cover surface 141 and the bottom surface 142 may form the upper surface and the lower surface of the support tube assembly 1, and may be arranged in series with any one of the front surface 110, the rear surface 120, the first side surface 130, and the second side surface 140. For example, the cover surface 141 may be arranged in series with the front surface 110, the bottom surface 142 may be arranged in series with the rear surface 120, or they may also be arranged in series with the same surface. Because the front surface 110, the rear surface 120, the first side surface 130, and the second side surface 140 are folded with respect to each other to form a polygonal pillar shape, the cover surface 141 and the bottom surface 142 form the upper surface and the bottom surface of the support tube assembly 1 through the folding process simply by being arranged in series with any one of the front surface 110, the rear surface 120, the first side surface 130, and the second side surface 140.

A cover adhesive surface 141a is formed on one side of the cover surface 141, a bottom adhesive surface 142a is formed on one side of the bottom surface 142, and the adhesive surfaces are folded to be adhered to other adjacent surfaces, thereby forming the foldable support tube 100.

The cover surface 141 and the bottom surface 142 cover the upper and lower portions of the foldable support tube 100 to prevent external foreign substances from entering the inside of the foldable support tube 100.

The foldable support tube 100 in a packaged state in which the cover surface 141 and the bottom surface 142 are folded and the respective cutting lines are not cut may be provided to a medical professional, and at the time of the procedure, after an open path cutting line 112, a side lower cutting line 113, a cutting line for a gel insertion hole cover 116, etc. are cut and the cover surface 141 and the bottom surface 142 are removed, the foldable support tube 100 is used.

The cutting line for the gel insertion hole cover 116 that is cut to communicate with a space inside the gel accommodation space 150 is formed at a corresponding position between the position of the upper end of an open path and the cover surface 141 on any one of the front surface 110, the rear surface 120, the first side surface 130, and the second side surface 140. If the gel insertion hole cover cutting line is cut, an opening (gel insertion hole) of the gel accommodation space 250 is located at the cut-off portion, and a user may inject the gel into the gel accommodation space 250 through the opening.

A first separation cutting line 114 extends from the open path cutting line 112 to the gel insertion hole cutting line, and a second separation cutting line 115 extends from the gel insertion hole cutting line to a connection portion connected to the cover surface 141. The first separation cutting line 114 and the second separation cutting line 115 are cut and used when the Foley catheter 10 is separated from the foldable support tube 100, after inserting the Foley catheter 19 into the bladder.

A lower adhesive surface and an upper adhesive surface may be provided to have a certain area at the lower and upper ends of any one of the front surface 110, the rear surface 120, the first side surface 130, and the second side surface 140. FIG. 2 illustrates that a lower adhesive surface 142a and an upper adhesive surface 141a are provided at the lower and upper ends of the second side surface 140. The lower adhesive surface 142a and the upper adhesive surface 141a are folded with respect to the second side surface 140, and are adhered to the bottom surface 142 and the cover surface 141 when assembled into the foldable support tube 100, as shown in FIG. 11, so that the shape of the foldable support tube 100 is maintained. In addition, a side adhesive surface 117 is provided at the end of the front surface 110, and is capable of being adhered to the rear surface 120 when folded and assembled into the foldable support tube 100. However, this is only an example of formation of the adhesive surfaces, and embodiments in which a lower adhesive surface and an upper adhesive surface are provided to have a certain area on any one of the four surfaces 110, 120, 130, and 140 are also possible.

The first roller 310 and the second roller 320 to transport the catheter tube 12 may be coupled to the foldable support tube 100 formed by folding the front surface 110, the rear surface 120, the first side surface 130, and the second side surface 140.

That is, a state shown in FIG. 13 may be prepared by assembling the first roller 310 and the second roller 320 with the foldable support tube 100 in the folded state (see FIG. 11).

For this purpose, the pair of first roller coupling holes 123 and 143 into which the first roller 310 is inserted may be formed through one of the front surface 110, the rear surface 120, the first side surface 130, and the second side surface 140, and another facing the surface, and the pair of second roller coupling holes 124 and 144 into which the second roller 320 is inserted may be formed at positions spaced apart from the pair of first roller coupling holes 123 and 143 in the lateral direction (width direction).

The positions of the corresponding surfaces through which the first roller coupling holes 123 and 143 and the second roller coupling holes 124 and 144 are formed may be lower than the position of the gel accommodation space 250.

The first roller 310 is coupled to the first roller coupling holes 123 and 143, as shown in FIG. 11. A pressing cutting line 143a is formed in an oval shape around the first roller coupling hole 143. When the first roller 310 is assembled and rotated to transport the catheter tube 12, the pressing cutting line 143a is pressed and torn by the rotational pressure of the first roller 310 to properly compress the catheter tube 12 and support the catheter tube 12 so that the catheter tube 12 is smoothly inserted into the person receiving the procedure.

At this time, the first roller coupling holes 123 and 143 may be provided with a reverse rotation prevention structure configured to prevent the first roller 310 from rotating in the reverse direction.

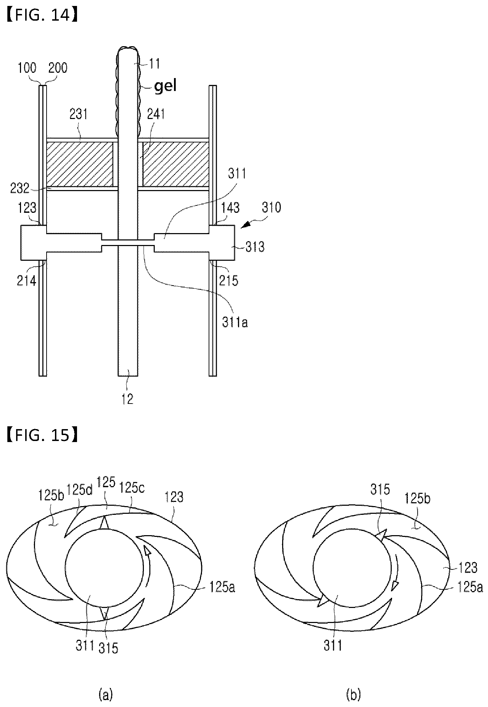

FIG. 15 is an exemplary view illustrating the reverse rotation prevention structure. As shown in FIG. 15, in order to prevent reverse rotation, unevennesses 125 and 145 are formed in the rotating direction of the first roller 310 on the inner circumferential surfaces of the first roller coupling holes 123 and 143. The unevennesses 125 and 145 are formed as guide surfaces 125a formed in the rotating direction of the first roller 310 to have a predetermined length on the inner surfaces of the first roller coupling holes 123 and 143. Here, a protrusion insertion recess 125b is provided between adjacent guide surfaces 125a so that a reverse rotation prevention protrusion 315 protruding from the outer circumferential surface of the first roller 310 is inserted thereinto when the first roller 310 rotates in the reverse direction.

Furthermore, the unevennesses 125 and 145 include a first uneven surface 125c that is curved with a first curvature in the circumferential direction from the inner circumferential surface of the corresponding first roller coupling hole, and a second uneven surface 125d that is curved with a second curvature different from the first curvature in the circumferential direction from the inner circumferential surface and is connected to the first uneven surface at one point (see FIG. 15).

The second roller 320 is inserted into the first roller coupling holes 124 and 144, and the catheter tube 12 is located in a space between the first roller 310 and the second roller 320 so that one side thereof may be in contact with the first roller 310 and the other side thereof may be in contact with the second roller 320. That is, if the first roller 310 is rotated, the opposite side of the catheter tube 12 that is in contact with the first roller 310 may be supported by the second roller 320 and move forward in conjunction with rotation of the first roller 310.

The first roller 310 and the second roller 320 may be provided separately from the foldable support tube 100, as described above, after the process of folding the foldable support tube 100 has been performed, assembly of the driving module 200 may be performed and then the driving module 200 may assembled with the foldable support tube 100.

That is, the first roller 310 and the second roller 320 are connected to the first roller coupling holes 123 and 143 and the second roller coupling holes 124 and 144 in the folded state of the foldable support tube 100, as shown in FIG. 11.

The first roller includes the roller main body 311 that guides transport of the catheter tube 12, and an operation knob 313 coupled to the roller main body 311 to be rotated by a user by hand. The roller main body 311 is provided separately from the operation knob 313.

The roller main body 311 has an outer diameter that may be inserted into the first roller coupling holes 123 and 143. Here, the reverse rotation prevention protrusions 315 that protrude radially outward may be provided on the outer circumferential surface of an area of the roller main body 311 that is inserted into the first roller coupling holes 123 and 143.

As shown in FIG. 15(a), if a user rotates the operation knob 313 in a regular direction in which the catheter tube 12 is inserted into the bladder, the reverse rotation prevention protrusions 315 of the roller main body 311 are guided and move along the protrusion guide surfaces 125a of the unevenness 125. On the other hand, if the roller main body 311 is rotated in the reverse direction, i.e., a direction in which the catheter tube 12 moves away from the bladder, by external force, the reverse rotation prevention protrusions 315 are inserted into the protrusion insertion recesses 125b and caught between neighboring portions of the unevenness 125. Thereby, reverse movement of the catheter tube 12 may be prevented, and separation of the catheter tube 12 from the bladder may be prevented.

The first roller 310 and the second roller 320 may be spaced apart from to correspond to the diameter of the catheter tube 12, and when the user rotates the operation knob 313 to rotate the first roller 310. The second roller 320 gear-coupled to the first roller 310 rotates within the second roller coupling holes 124 and 144, and the catheter tube 12 disposed between the first roller 310 and the second roller 320 moves forward or backward due to frictional force with the rollers.

The above-described driving module 200 may be inserted into the foldable support tube 100 from which the cover surface 141 and the bottom surface 142 have been removed. More specifically, as shown in FIGs. 12 and 13, the driving module 200 may be inserted into a position where the first roller insertion holes 214 and 215 of the driving module 200 are aligned with the first roller coupling holes 123 and 143 of the foldable support tube 100, and the second roller insertion holes 223 and 224 of the driving module 200 are aligned with the second roller coupling holes 124 and 144 of the foldable support tube 100.

When assembly of the foldable support tube 100 and the driving module 200 is completed, it is possible to inject the gel into the gel accommodation space 250 through the opening and insert the Foley catheter 10 into the support tube assembly 1 to perform the procedure.

The foldable support tube 100 may be formed by folding or adhering the front surface 110, the rear surface 120, the first side surface 130, the second side surface 140, the cover surface 141, and the bottom surface 142, which are described above, and the support tube assembly having the shape shown in FIG. 13 may be formed by coupling the driving module 200, the first roller 310, and the second roller 320 to the foldable support tube 100.

However, before being actually used in the procedure, specifically during the assembly process of the foldable support tube 100 and the driving module 200 and the pre-insertion process of the Foley catheter, the opening to inject the gel into the gel accommodation space, the opening to insert the Foley catheter 10 into the foldable support tube 100, etc. are required. Therefore, in the present invention, separate cutting lines are formed on the foldable support tube 100 provided in the form of the development view, and the openings may be formed by cutting some components along the cutting lines before use.

The open path cutting line 112 may be formed in the length direction from the bottom of the support tube to a position spaced apart from the bottom of the gel accommodation space 250 toward the bottom of the support tube by a predetermined distance.

That is, when the development view is folded, the open path cutting line 113 is not cut so that the open path is maintained in an unopened state, as shown in FIG. 11, and when a medical professional cuts the open path cutting line 112 before use, the open path is opened, as shown in FIG. 12.

At this time, the lower cutting line 113 that is disposed to be inclined toward the surfaces on both sides of the surface where the open path cutting line 112 is formed are provided at the bottom of the open path. Because the lower cutting line 113 is disposed to be inclined, the lower end of the Foley catheter is exposed to the outside, and is easily connected to a urine bag. The lower cutting line 113 is separated from the front surface 110, the rear surface 120, the first side surface 130, and the second side surface 140 together with the open path, and the lower part of the foldable support tube is opened to secure a space into which the Foley catheter 10 may be inserted.

A process of using the support tube assembly 1 according to one embodiment of the present invention having the above configuration will be described with reference to the accompanying drawings.

As shown in FIG. 13, the support tube assembly 1 according to one embodiment of the present invention includes the foldable support tube 100 formed by folding the developed form thereof, the driving module 200, and the first roller 310 and the second roller 320 coupled thereto.

The surface of the foldable support tube 100 may be waterproof-coated, the Foley catheter 10 is inserted into the foldable support tube 100 in the folded state, the first roller 310 and the second roller 320 are coupled to the first roller coupling holes 123 and 143 and the second roller coupling holes 124 and 144, and the first roller insertion holes 214 and 215 and the second roller insertion holes 223 and 224, and the foldable support tube 100 is packaged in an antibacterial and sealing manner and then provided to a medical professional, etc.

At the manufacturing site of the foldable support tube 100, the foldable support tube 100 may be manufactured by folding the developed form thereof. A worker folds the front surface 110, the rear surface 120, the first side surface 130, and the second side surface 140 along the folding lines of the development view shown in FIG. 10 to form the support tube into a rectangular tube shape.

In addition, the rectangular foldable support tube 100 is manufactured by bonding the respective adhesive surfaces, as shown in FIG. 11, and the driving module 200 is installed in the foldable support tube 100. The manufactured support tube assembly 1 in a state in which the Foley catheter 10 is installed may be sterilized and packaged, and then be provided to a medical professional.

When the medical professional needs to insert the Foley catheter 10 into a patient, the medical professional opens the package of the support tube assembly 1, and cuts the open path cutting line 112 and the lower cutting line 113 of the foldable support tube 100, as shown in FIG. 12, so that the lower part and the open path of the foldable support tube 100 are exposed to the outside.

The medical professional injects a gel into the gel accommodation space 250 through the opening of the gel accommodation space 250, and inserts the Foley catheter 10 from the lower part to the upper part of the foldable support tube 10.

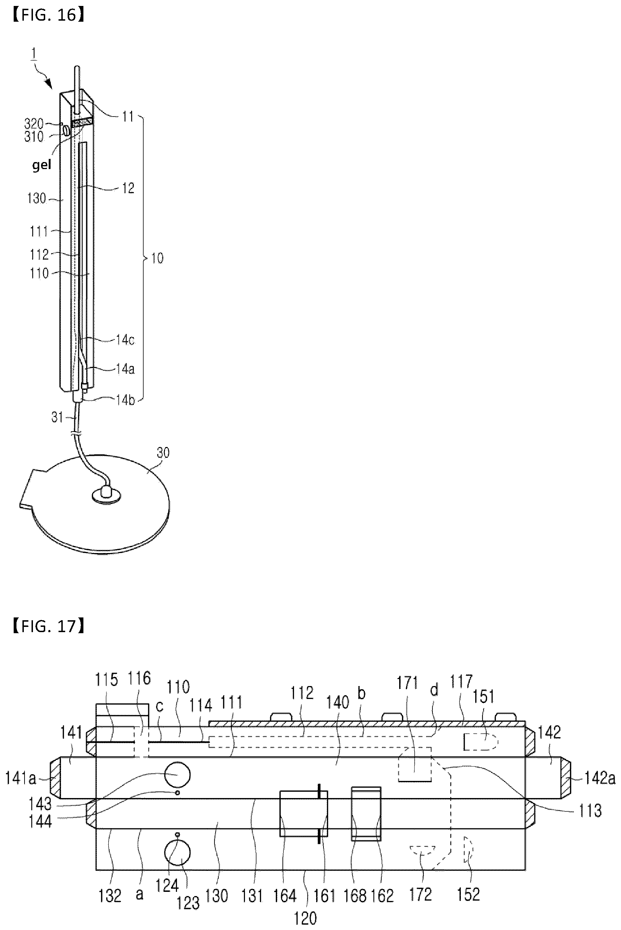

As shown in FIG. 16, the insertion tip 11, the catheter tube 12, and branch tubes 14 of the Foley catheter 10 are sequentially inserted into the opened lower part of the foldable support tube 100, and the insertion tip 11 is moved upward via the gel accommodation space 250 by operating the operation knob 313 in a regular direction by the medical professional.

At this time, if the operation knob 313 is operated in the regular direction, the main roller 310 rotates stably along the guide surfaces 125a and 145a in the first roller coupling holes 123 and 143, and if the operation knob 313 is operated in the reverse direction or the first roller 310 rotates in the reverse direction due to external force, the reverse rotation prevention protrusions 315 are caught in the insertion recesses 125b to prevent reverse rotation of the first roller 310.

When the catheter tube 12 is transported and the insertion tip 11 is inserted into the bladder, as shown in FIG. 6, the Foley catheter 10 is separated from the support tube assembly 1 by tearing the first separation cutting line 114 and the second separation cutting line 115 and opening the driving module 200 so that the third parts move away from each other.

FIG. 17 is a development view of a foldable support tube according to another embodiment of the present invention.

Hereinafter, a description of the same parts as those of the folding support tube 100 according to the embodiment of FIG. 10 will be omitted, and a description will be focused on parts that are different from the same.

The foldable support tube according to embodiment of FIG. 17 is provided so that a syringe 400 may be accommodated in a support tube assembly 1. For this purpose, a first cylinder accommodation part cutting line 161 is formed in the lateral direction (width direction) of the foldable support tube 100 on the remaining surfaces except for a surface on which an open path cutting line 112 is formed, among a front surface 110, a rear surface 120, a first side surface 130, and a second side surface 140, and a second cylinder accommodation part cutting line 164 is formed in the lateral direction at a portion spaced apart from the first cylinder accommodation part cutting line 161 by a predetermined distance in the longitudinal direction. As the first cylinder accommodation part cutting line 161 and the second cylinder accommodation part cutting line 164 are cut and connection portions between the first cylinder accommodation part cutting line 161 and the second cylinder accommodation part cutting line 164 are folded toward the inside of the foldable support tube 100, a cylinder insertion space N in which a cylinder 410 of the syringe 400 is accommodated may be formed.

In addition, a first piston accommodation cutting line 168 is formed in the lateral direction at a portion spaced apart from the first cylinder accommodation part cutting line 161 by a predetermined distance in the opposite direction to the second cylinder accommodation part cutting line 164, and a second piston accommodation cutting line 162 is formed in the lateral direction at a portion spaced apart from the first piston accommodation cutting line 168 by a predetermined distance in the longitudinal direction. As the first piston accommodation cutting line 168 and the second piston accommodation cutting line 162 are cut and connection portions between the first piston accommodation cutting line 168 and the second piston accommodation cutting line 162 are folded toward the inside of the foldable support tube 100, a piston insertion space M in which a piston 420 of the syringe 400 is accommodated may be formed.

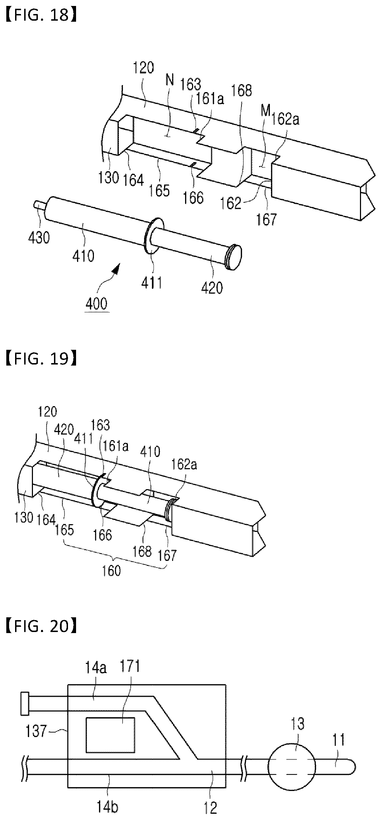

Referring to FIG. 18, a syringe accommodation part 160 forms the cylinder insertion space N into which the cylinder 410 of the syringe 400 is inserted and the piston insertion space M into which the piston 420 is inserted.

The cylinder insertion space N is a space that may accommodate the cylinder 410 of the syringe 400, prepared by cutting the first cylinder accommodation part cutting line 161 and the second cylinder accommodation part cutting line 164 and folding the connections portions on the front surface 110, the rear surface 120, the first side surface 130, and the second side surface 140 between the first cylinder accommodation part cutting line 161 and the second cylinder accommodation part cutting line 164 toward the inside of the foldable support tube 100.

The piston insertion space M is a space that may accommodate the piston 420 of the syringe 400, prepared by cutting the first piston accommodation cutting line 168 and the second piston accommodation cutting line 162 and folding the connections portions on the front surface 110, the rear surface 120, the first side surface 130, and the second side surface 140 between the first piston accommodation cutting line 168 and the second piston accommodation cutting line 162 toward the inside of the foldable support tube 100.

Flange insertion cutting lines 163 and 166 extend outward in the lateral direction from the connection portions of the cylinder insertion space N or the piston insertion space M that are folded toward the inside of the foldable support tube 100, and a space into which a flange 411 of the syringe 400 may be inserted is prepared by cutting the flange insertion cutting lines 163 and 166, so that the syringe 400 may be fixed within the syringe accommodation part 160. The accompanying drawings illustrate that the flange insertion cutting lines 163 and 166 are formed in the cylinder insertion space N, but it should be understood that the flange insertion cutting lines are not limited thereto and may be formed in the piston insertion space M.

The syringe 400 is coupled to a fluid injection tube 14a to supply a fluid, such as distilled water, thereby inflating a balloon 13 connected to the fluid injection tube 14a so as to transfer the fluid therebetween. The inflation process of the balloon 13 may be performed after insertion of the insertion tip 11 into the bladder, and as the balloon 13 is inflated, the insertion tip 11 is capable of being maintained in the bladder.

Furthermore, the foldable support tube according to this embodiment of the present invention is different from that of the former embodiment in that components configured to form a medical supplies accommodation part capable of storing separate medical supplies are further provided.

A medical supplies accommodation space formation surface 151 is formed at a position of any one of the front surface 110, the rear surface 120, the front side surface 130, and the second side surface 140, which is lower than the lower cutting line 113, and an insertion recess 152 is formed on a surface facing the surface provided with the medical supplies accommodation space formation surface 151.

A part of the medical supplies accommodation space formation surface 151 is provided with a cutting line, and when cutting along the cutting line is completed, the medical supplies accommodation space formation surface 151 acts as a partition wall that traverses the inner space of the foldable support tube 100. A medical supplies accommodation space from the bottom of the foldable support tube 100 to the medical supplies accommodation space formation surface 151 is formed by inserting the cut part of the medical supplies accommodation space formation surface 151 into the insertion recess 152. Medical supplies necessary for the Foley catheter procedure, such as disinfectant cotton, a gel, and distilled water, may be stored in the medical supplies accommodation space.

A backward movement prevention part configured to prevent the Foley catheter 100 from moving backward is applied to the foldable support tube 100 according to another embodiment of the present invention. This will be described in detail with reference to FIGs. 10 and 18.

The insertion tip 11 of the Foley catheter 10 may be inserted into the bladder and the balloon 13 may be inflated so that the catheter tube 12 may be fixed while being inserted into the bladder, and this is because the diameter of the inflated balloon 13 is greater than the diameter of the urethra and thus the balloon 13 serves as a stopper. Therefore, if the balloon 13 is deflated by discharging the fluid in the balloon 13 to the outside, the Foley catheter 10 may move backward.

In order to solve this problem, in the present invention, the backward movement prevention part arranged in parallel with any one of the front surface 110, the rear surface 120, the front side surface 130, and the second side surface 140 may be additionally provided (see FIG. 10).

The configuration of the backward movement prevention part is similar to the principle of forming the medical supplies accommodation space.

A backward movement prevention part formation surface 171 is provided at a position of any one of the front surface 110, the rear surface 120, the front side surface 130, and the second side surface 140, which is higher than the lower cutting line 113, and an insertion recess 172 is formed on a surface facing the surface provided with the backward movement prevention part formation surface 171.

A part of the backward movement prevention part formation surface 171 is provided with a cutting line, and when cutting along the cutting line is completed and the cut part of the backward movement prevention part formation surface 171 is inserted into the insertion recess 172, the backward movement prevention part formation surface 171 acts as a partition wall that traverses the inner space of the foldable support tube 100.

The backward movement prevention part formation surface 171 traverses the inner space of the foldable support tube 100, thereby being capable of preventing the Foley catheter 10 from moving backward.

FIG. 20 illustrates that the backward movement prevention part formation surface 171 prevents the Foley catheter 10 from moving backward. The branch tubes 14 include the fluid injection tube 14a and a discharge tube 14b that are branched from one end of the catheter tube 12, and in one example, the fluid injection tube 14a includes a portion that extends with a designated slope from the branched area, and a portion that is connected to the extending portion and extends parallel to the discharge tube 14b. Because the backward movement prevention part formation surface 171 traverses the inner space of the foldable support tube 100, the branched tubes (branch tubes) of the Foley catheter 10 are caught on the backward movement prevention part formation surface 171, thereby preventing the Foley catheter 10 from moving backward. After the procedure, the backward movement prevention part formation surface 17 may be cut off, and thereby, the Foley catheter 10 may be easily separated from inside of the foldable support tube 100.

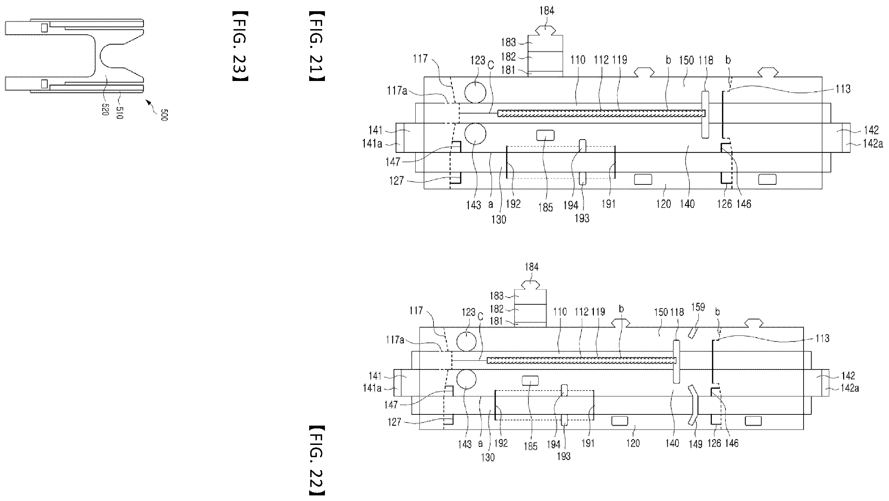

Referring to FIG. 21, a foldable support tube according to yet another embodiment of the present invention will be described.

The foldable support tube according to FIG. 21 is characterized in that i) an upper cutting line 117 configured to open the upper part of the foldable support tube is separately formed, ii) a syringe accommodation part configured to both a cylinder and a piston is formed and syringe cover surfaces 181, 182, 183, and 184 configured to cover the outer surface of a syringe accommodated in the syringe accommodation part are further formed, iii) support cutting lines 126, 127, 146, and 147 configured to form supports to support the foldable support tube after cutting are further formed, and iv) in order to facilitate cutting of an open path cutting line 112, an open path exposure cutting line 118 configured to expose a part of the open path cutting line 112 is further formed, and incision parts 119 are further formed.

The upper cutting line 117 is formed to traverse a front surface 110, a rear surface 120, a first side surface 130, a second side surface 140, and an attachment surface 150 in the width direction. If parts of the surfaces 110, 120, 130, 140, and 150 are cut along the upper cutting line 117, the upper part of the foldable support tube is opened. In addition, the above-descried driving module 200 is installed through the opened upper part.

Furthermore, the upper cutting line 117 includes parts 117a that extend in the length direction of some of the surfaces 110, 120, 130, 140, and 150. By forming the parts 117a that extend in the length direction, the upper cutting line 117 may be more easily cut.

Further, a first syringe accommodation part cutting line 191 is formed in the width direction of the foldable support tube 100 on the remaining surfaces except for a surface on which the open path cutting line 112 is formed, among the front surface 110, the rear surface 120, the first side surface 130, and the second side surface 140, and a second syringe accommodation part cutting line 192 is formed in the lateral direction at a portion spaced apart from the first syringe accommodation part cutting line 191 by a predetermined distance in the longitudinal direction. As the first syringe accommodation part cutting line 191 and the second syringe accommodation part cutting line 192 are cut and connection portions between the first syringe accommodation part cutting line 191 and the second syringe accommodation part cutting line 192 are folded toward the inside of the foldable support tube 100, a space in which the syringe 400 is accommodated may be formed.

Flange insertion cutting lines 193 and 194 into which a flange of the cylinder 410 is inserted is formed between the first syringe accommodation part cutting line 191 and the second syringe accommodation part cutting line 192.

Furthermore, the foldable support tube 100 according to FIG. 21 has the syringe cover surfaces 181, 182, 183, and 184 that cover a portion of the outer surface of the syringe accommodation part after the syringe 400 has been accommodated in the syringe accommodation part.

The syringe cover surfaces 181, 182, 183, and 184 may be formed to be sequentially arranged in the width direction on the side of the attachment surface 150, and connection portions between the respective surfaces are folded so that the syringe cover surface 184 is inserted into a syringe cover surface insertion space 185, thereby being capable of surrounding the portion of the outer surface of the syringe accommodation part. Therefore, the syringe 400 accommodated in the syringe accommodation part is supported by the syringe cover surfaces 181, 182, 183, and 194 from the outside.

Furthermore, the support cutting lines 126, 127, 146, and 147 are formed to be bent so that one side of each thereof is connected to a side lower cutting line 113 or the upper cutting line 117 and the other side of each thereof is connected to a corresponding one of the connection folding lines between the first side surface 130, and the rear surface 120 and the second side surface 140. Therefore, after cutting the side lower cutting line 113 and the upper cutting line 117, when the support cutting lines 126, 127, 146, and 147 are cut, the supports serving as legs of the foldable support tube 100 may be formed. Therefore, the support stability of the foldable support tube 100 is increased.

The open path exposure cutting line 118 extends in the width direction from the attachment surface 150 to the front surface 110 and the second side surface 140 so that a portion (rear end) of the open path cutting line 112 is exposed, and has a predetermined width.

As shown in FIG. 21, the rear end of the open path cutting line 112 is located in (i.e., exposed to) a space formed by cutting the open path exposure cutting line 118, and when a user applies force toward the upper end of the open path cutting line 112 while holding a portion of the exposed open path cutting line 112, the open path cutting line 112 is cut from the front surface 110. In order to ensure ease of cutting, the incision parts 119 are formed in an oblique direction inside the open path cutting line 112, and the oblique direction in which the incision parts 119 are formed may be opposite to the cutting direction. Therefore, it is possible to provide support during the process of cutting the open path cutting line 112.

Further, referring to FIGs. 22 and 23, a foldable support tube according to still yet another embodiment of the present invention will be described.

Referring to FIG. 22, a clip cutting line 149 may be formed on a rear surface 120, a first side surface 130, and a second side surface 140. The clip cutting line 149 formed on the first side surface 130 may be formed to traverse the first side surface 130, and the clip cutting line 149 formed on the rear surface 120 and the second side surface 140 may be formed to be inclined in a direction approaching the front end of the foldable support tube as it becomes far away from the clip cutting line on the first side surface 130.

In addition, a clip cutting line 159 according to this embodiment of the present invention may be additionally formed on an attachment surface 150, and may have the same shape as the clip cutting line 149 formed on the rear surface 120 or the second side surface 140, so that when the respective surfaces are folded to form the foldable support tube, the clip cutting line 159 may partially overlap the clip cutting line 149.

After cutting the clip cutting lines 149 and 159, a clip 500 is installed in the cut-off portions. Referring to FIG. 23, the clip 500 includes a first body 510 and a second body 520. In one example, the clip 500 may be manufactured using a synthetic resin having a predetermined strength, such as plastic.

The first body 510 and the second body 520 are connected to each other at the rear ends thereof, and the front ends thereof are spaced apart from each other by a predetermined interval. The spaced portions of the first and second bodies are inserted into the cut-off portions to be installed in the foldable support tube.

A portion of a Foley catheter 10 is exposed to the outside through the open path of the foldable support tube, a urine bag and a syringe are coupled to the rear end of the Foley catheter 10, and due to the characteristics of the foldable support tube formed of paper, the shape of the foldable support tube may be deformed by the weight of the Foley catheter 10, the urine bag, and the syringe. Therefore, in the present invention, the clip 500 is installed to prevent the open path from widening and support the weight of the Foley catheter, etc., thereby being capable of securing accuracy and ease of the procedure.

The present invention, as described above, has the following effects.

A foldable support tube may be manufactured using a foldable material (paper, vinyl, or a synthetic resin material) while providing the same function as conventional Foley catheter support tubes, thereby being capable of reducing manufacturing costs.

A driving module that provides driving force to guide a Foley catheter is manufactured using a material having predetermined rigidity, and is installed in the foldable support tube, thereby durability of a support tube assembly and improving accuracy of a procedure.

The support tube assembly in a state in which the upper and lower parts of the support tube assembly are closed by a bottom surface and a cover surface before use is provided to a medical professional, and the bottom surface and the cover surface are removed just before the procedure is performed on a person receiving the procedure, thereby being capable of being easily stored and transported, and maintaining sterility for a long period of time.

A reverse rotation prevention structure for transportation rollers that guide the Foley catheter into the bladder is applied, thereby preventing the Foley catheter from being separated between a main roller and a sub-roller during storage, and solving separation of the Foley catheter inserted into the body of the person receiving the procedure from the body at the time of the procedure.

In addition, a backward movement prevention part configured to prevent the Foley catheter from moving backward is applied, thereby preventing the Foley catheter from being separated between the main roller and the sub-roller during storage, and solving separation of the Foley catheter inserted into the body of the person receiving the procedure from the body due to backward movement of the Foley catheter at the time of the procedure.

In addition, a medical supplies accommodation part that may store various medical supplies (a syringe, disinfectant cotton, a gel, distilled water, etc.) required during the procedure of inserting the Foley catheter into the bladder are provided, thereby enabling the procedure to be simply performed without a process of preparing separate medical supplies required for the procedure by a medical professional.

In addition, the lower end of the Foley catheter is supported by a fourth part and a fifth part, and the upper end of the Foley catheter is pressed by a pressing element, thereby enabling the Foley catheter to be inserted into a correct position without being deflected in every direction.

In addition, a clip having a predetermined strength is installed on the foldable support tube, thereby being capable of preventing the shape of the foldable support tube from being deformed due to the weight of components required for the Foley catheter procedure.

As described above, the present invention has been described with reference to the embodiments illustrated in the drawings so that those skilled in the art can easily understand and reproduce the present invention, but the embodiments are merely exemplary and it will be apparent to those skilled in the art that various modifications and equivalent other embodiments are possible from the embodiments of the present invention. Accordingly, the scope of protection of the present invention should be defined by the claims.

### (Description of reference numerals and marks)

1: Support tube assembly
10: Foley catheter
11: Insertion tip
12: Catheter tube
13: Balloon
14: Branch tube
14a: Fluid injection tube
14b: Discharge tube
14c: Connection tube
30: Urine storage pack
100: Foldable support tube
110: Front surface
111: Folding line
112: Open path cutting line
113: Lower cutting line
114: First separation cutting line
115: Second separation cutting line
116: Gel insertion hole cover
117: Upper cutting line
118: Open path exposure cutting line
119: Incision part
120: Rear surface
123: First roller coupling hole
124: Second roller coupling hole
125: Unevenness
125a: Guide surface
125b: Protrusion insertion recess
126, 127: Support cutting line
130: First side surface
131, 132: Folding line
140: Second side surface
141: Cover surface
141a: Cover adhesive surface
142a: Bottom adhesive surface
142: Bottom surface
143: First roller coupling hole
143a: Pressing cutting line
144: Second roller coupling hole
145: Unevenness
145a: Guide surface
146, 147: Support cutting line
149: Clip cutting line
150: Attachment surface
151: Medical supplies accommodation space formation surface
152: Insertion recess
159: Clip cutting line
160: Syringe accommodation part
161: First cylinder accommodation part cutting line
162: Second piston accommodation cutting line
163: First flange insertion cutting line
164: Second cylinder accommodation part cutting line
166: Second flange insertion cutting line
168: First piston accommodation cutting line
171: Backward movement prevention part formation surface
172: Insertion recess
181, 182, 183, 184: Syringe cover surface
185: Syringe cover surface insertion space
191: First syringe accommodation part cutting line
192: Second syringe accommodation part cutting line
193, 194: Flange insertion cutting line
200: Driving module
210: First part
211, 212: Side surface
213: Connection surface
214, 215: First roller insertion hole
220: Second part
223, 224: Second roller insertion hole
230: Third part
231: Upper surface
232: Lower surface
233, 234: Half guide hole
235, 236: Leakage prevention part
237: Coupling part
240: Fourth part
241: Guide groove
250: Gel accommodation space
260: Fifth part
261: Groove
271: Insertion protrusion
272: Insertion groove
280: Pressing element
310: First roller
311: Roller main body
313: Operation knob
315: Reverse rotation prevention protrusion
320: Second roller
321: Roller main body
321a, 321b: Alignment groove
400: Syringe
410: Cylinder
420: Piston
500: Clip
510: First body
520: Second body
A: Genitalia
B: Bladder
C: Gel
N: Cylinder accommodation space
M: Piston accommodation space

## Claims

1. A driving module for Foley catheter support assemblies installed in a support tube configured to guide a Foley catheter when inserting the Foley catheter, comprising:
a first part comprising side surfaces provided with a pair of first roller insertion holes configured such that a first roller is installed therein, and a connection surface between the side surfaces;
a second part disposed on the connection surface of the first part aligned with the pair of first roller insertion holes, and provided with a pair of second roller insertion holes configured such that a second roller is installed therein;
third parts disposed on the side surfaces of the first part, respectively, and provided with a pair of half guide holes configured to guide the Foley catheter; and
a fourth part disposed on the connection surface of the first part so as to be located within a space formed by the third parts.

2. The driving module according to claim 1, wherein a distance from the connection surface of the first part to the first roller insertion holes is longer than a distance from the connection surface of the first part to the second roller insertion holes.

3. The driving module according to claim 2, wherein a difference between the distance from the connection surface of the first part to the first roller insertion holes and the distance from the connection surface of the first part to the second roller insertion holes corresponds to a thickness of the Foley catheter.

4. The driving module according to claim 1, wherein each of the third parts comprises an upper surface provided with one of the pair of half guide holes, and a lower surface spaced apart from the upper surface and provided with the other of the pair of half guide holes.

5. The driving module according to claim 4, wherein a direction in which the pair of first roller insertion holes of the first part face each other and a direction in which the pair of half guide holes of the third parts face each other intersect each other.

6. The driving module according to claim 4, wherein a jelly accommodation space surrounded by the upper surface and the lower surface of each of the third parts and the side surfaces of the first part is formed by folding connection portions between the side surfaces and the connection surface of the first part in a direction in which the third parts approach each other.

7. The driving module according to claim 6, wherein leakage prevention parts are formed on contact surfaces of the third parts provided on the side surfaces of the first part in a longitudinal direction of the contact surfaces.

8. The driving module according to claim 7, wherein a coupling part is formed on at least one of the leakage prevention parts so as to be coupled to the other of the leakage prevention parts.

9. The driving module according to claim 6, wherein the fourth part has a predetermined height and is disposed in the jelly accommodation space.

10. The driving module according to claim 9, wherein a guide groove configured to extend in an insertion direction of the Foley catheter is formed on the fourth part.

11. The driving module according to claim 4, wherein the half guide hole formed on the upper surface is disposed closer to the connection surface of the first part than the half guide hole formed on the lower surface.

12. The driving module according to claim 4, wherein the half guide hole formed on the lower surface is disposed closer to the connection surface of the first part than the half guide hole formed on the upper surface.

13. The driving module according to claim 1, wherein:
gear teeth are formed on an outer surfaces of the first roller and the second roller; and
an alignment groove is formed on the outer surface of the first roller or the second roller.

14. The driving module according to claim 13, wherein the alignment groove has a depressed portion from the outer surface of the first roller or the second roller in a direction away from the Foley catheter.

15. The driving module according to claim 14, wherein a distance from the first roller or the second roller to the alignment groove corresponds to a thickness of the Foley catheter.

16. The driving module according to claim 13, wherein the Foley catheter is manufactured using a soft material.

17. A Foley catheter support tube assembly provided with the driving module according to any one of claims 1 to 16 installed therein, comprising:
a front surface, a rear surface, a first side surface, and a second side surface,
wherein:
an open path configured such that a fluid injection tube of the Foley catheter passes therethrough is formed in any one of the front surface, the rear surface, the first side surface, and the second side surface; and
a pair of first roller coupling holes aligned with the pair of first roller insertion holes of the first part is formed in surfaces located on both sides of the surface provided with the open path formed therein.

18. The Foley catheter support tube assembly according to claim 17, wherein:
first and second syringe accommodation part cutting lines configured to traverse at least three surfaces other than the surface provided with the open path formed therein are further formed; and
syringe cover surfaces are formed to be sequentially arranged in parallel on one side of an attachment surface arranged parallel to the front surface.

19. The Foley catheter support tube assembly according to claim 18, wherein:
a syringe accommodation part is formed by cutting the first and second syringe accommodation part cutting lines and folding connection portions between the three surfaces; and
the syringe cover surfaces are folded to surround an outer surface of the syringe accommodation part.

20. The Foley catheter support tube assembly according to claim 17,
an upper cutting line and a side lower cutting line configured to traverse the front surface, the rear surface, the first side surface, and the second side surface are further formed; and
on both side surfaces connected to a surface opposite to the surface provided with the open path formed therein, first support cutting lines bent so that one side of each thereof is connected to the upper cutting line and the other side of each thereof is connected to a corresponding one of connection portions between the opposite surface and the side surfaces, and second support cutting lines bent so that one side of each thereof is connected to the side lower cutting line and the other side of each thereof is connected to a corresponding one of the connection portions between the opposite surface and the side surfaces are formed.

21. The Foley catheter support tube assembly according to claim 20, wherein supports configured to support the support tube assembly are formed by cutting the upper cutting line, the side lower cutting line, the first support cutting lines, and the second support cutting lines.

22. The Foley catheter support tube assembly according to claim 17, wherein:
an open path cutting line cut to form the open path is formed on any one of the front surface, the rear surface, the first side surface, and the second side surface, and
an open path exposure cutting line configured to expose a portion of the open path cutting line is further formed on the surface.

23. The Foley catheter support tube assembly according to claim 22, wherein:
incision parts are formed in an oblique direction inside the open path cutting line; and
the oblique direction is a direction inclined opposite to a direction in which the open path cutting line is cut.

24. The Foley catheter support tube assembly according to claim 22, wherein a clip cutting line configured to install a clip therein is formed on each of three surfaces other than the surface provided with the open cutting line formed thereon.
